**Europäisches Patentamt**

(19) **European Patent Office**

**Office européen des brevets**

(11) Numéro de publication: **0 099 812**
**B1**

(12) # FASCICULE DE BREVET EUROPÉEN

(45) Date de publication du fascicule du brevet: **29.04.87**

(51) Int. Cl.⁴: **G 01 N 33/569,** G 01 N 33/554

(21) Numéro de dépôt: **83401417.7**

(22) Date de dépôt: **08.07.83**

(54) Nouveau réactif de diagnostic des parasitoses et des allergies, procédé de préparation de ce nouveau réactif et procédé de diagnostic des parasitoses et des allergies à l'aide dudit réactif.

(30) Priorité: **09.07.82 FR 8212074**

(43) Date de publication de la demande:
**01.02.84 Bulletin 84/5**

(45) Mention de la délivrance du brevet:
**29.04.87 Bulletin 87/18**

(84) Etats contractants désignés:
**AT BE CH DE GB LI LU NL SE**

(73) Titulaire: **INSTITUT PASTEUR, 28, rue du Docteur Roux, F-75724 Paris Cedex 15 (FR)**

(72) Inventeur: **Leynadier, Francisque, 42 rue de Chaligny, F-75012 (FR)**

(74) Mandataire: **Ores, Irène, CABINET ORES 6, Avenue de Messine, F-75008 Paris (FR)**

(56) Documents cité:
FR-A-2 315 251
FR-A-2 346 717

CHEMICAL ABSTRACTS, vol. 79, no. 3, 23 juillet 1973, page 77, no. 14059u, Columbus, Ohio, USA, A. CHAGNON et al.: "Use of an organic buffer (HEPES) (N-2-hydroxyethylpiperazine-N'-2-ethanesulfonic acid) in human lymphocytoid cell line cultures"
CHEMICAL ABSTRACTS, vol. 91, no. 5, 30 juillet 1979, page 263, no. 35114w, Columbus, Ohio, USA, C.A. VEGA et al.: "Standardization of biological buffers in water-dimethyl sulfoxide mixtures"
THE JOURNAL OF IMMUNOLOGY, vol. 118, no. 4, avril 1977, pages 1317-1321, The Williams & Wilkins Co., USA, M.C. CONROY et al.: "Measurement of IgE on human basophils: relation to serum IgE and anti-IgE-induced histamine release"

(56) Documents cité: (suite)
CHEMICAL ABSTRACTS, vol. 89, no. 13, 25 septembre 1978, page 604, no. 105681p, Columbus, Ohio, USA, F.J. MALVEAUX et al.: "IgE receptors on human basophils. Relationship to serum IgE concentration"

EP 0 099 812 B1

**0 099 812**

## Description

La présente invention est relative à des prefectionnements apportés au procédé de préparation du réactif de diagnostic des parasitoses et des allergies qui fait l'objet de FR-A-2 460 481. Elle est plus particulièrement relative à des perfectionnements apportés aux milieux de séparation utilisés pour recueillir les cellules basophiles qui constituent ledit réactif.

Comme on le sait (cf. FR-A-2 460 481), la centrifugation d'un échantillon de sang prélevé chez un patient humain ou animal supposé allergique ou atteint d'une parasitose, provoque la séparation d'une couche leucocytaire et d'un plasma surnageant; après rejet du plasma, la couche leucocytaire est reprise par un tampon non destructeur à l'égard des cellules basophiles, avantageusement constitué par du tampon Hépès 1M (acide N-2-hydroxyéthylpipérazine-N'-2-éthane-sulfonique) ou Pipès (acide pipérazine-N,N'-bis-2-éthane-sulfonique), après quoi un liquide de densité 1,079-1,085 est injecté sous la suspension ainsi obtenue, pour permettre la séparation des basophiles et l'obtention finale du réactif de diagnostic constitué par une suspension riche en cellules basophiles. Une composition du tampon utilisé pour reprendre la couche leucocytaire, donnée à titre d'exemple dans le Brevet sus-indiqué, est la suivante:

Hépès, solution molaire 25 ml
KCl 10 % 0,932 ml
CaCl$_2$ 10 % 0,550 ml
MgCl$_2$ 10 % 0,510 ml
NaCl 9 ‰, (7,6 g) 844,11 ml
H$_2$0 distillée qsp 1000 ml
pH 7,4-7,6

Le liquide de densité 1,079-1,085 mis en oeuvre pour la séparation de scellules basophiles est défini comme présentant une osmolarité comprise entre 280 et 320 milliosmoles et étant avantageusement constitué par un mélange de métrizoate de sodium et de "Ficoll" (qui est une marque déposée désignant un polymère de saccharose et d'épichlorhydrine) ou de "Percoll" (qui est une marque déposée désignant une solution colloïdale de silice enrobée de polyvinylpyrrolidone) et ayant de préférence la composition suivante:

Métrizoate de sodium 15 g
"Ficoll" 12,867 g
Eau qsp 165,8 ml

Le FR-A-2 483 621 a proposé une composition quelque peu modifiée pour ledit milieu tampon, laquelle est la suivante, à titre d'exemple:

Hépès, solution molaire 4 à 25 ml
KCl 10 % 0,932 ml
CaCl$_2$ 10 % 0,550 ml
MgCl$_2$ 10 % 0,510 ml
NaCl 9 ‰ 850 à 900 ml
Eau qsp 1000 ml
pH 7,4-7,6,

la composition du liquide de densité utilisé pour la séparation des cellules basophiles n'étant pas modifiée par rapport au Brevet FR-A-2 460 481.

D'autre part, le FR-A-2 483 621 a proposé des perfectionnements au procédé de préparation du réactif de diagnostic des parasitoses et des allergies conforme au FR-A-2 460 481. Alors que selon le FR-A-2 460 481, la couche leucocytaire qui s'est séparée à la suite d'une opération de centrifugation effectuée sur du sang total, est reprise, après rejet du plasma, par un milieu tampon tel qu'indiqué ci-dessus, après quoi les cellules basophiles sont séparées au moyen d'un liquide de densité 1,079-1,085 injecté sous la suspension de leucocytes dans le milieu tampon, selon le FR-A-2 483 621, il est possible d'obtenir la séparation des basophiles sans avoir à procéder à la centrifugation préalable du sang total: en effet, la séparation des cellules basophiles au moyen du liquide de densité sous-jacent est effectuée à partir d'un mélange volume/volume de sang total et d'un tampon Hépès de composition appropriée contenant de l'héparine, le sang total ayant avantageusement été prélevé sur de l'éthylènediaminetétraacétate de potassium (EDTA K$_3$). Le tampon utilisé en pareil cas présente avantageusement la composition suivante:

Hépès, solution molaire 4 à 25 ml
KCl 10 % 0,932 ml
NaCl 9 ‰, 850 à 900 ml
Héparine 5 à 20 unités/ml
et de préférence 10 unités/ml
Eau qsp 1000 ml
pH 7,4-7,6

L'utilisation d'un tampon Hépès contenant de l'héparine a permis de supprimer une première étape de centrifugation destinée à séparer la couche leucocytaire du plasma pour la traiter par le liquide de densité en vue de la séparation des basophiles, et de simplifier, par conséquent, le procédé de séparation des basophiles en traitant directement un mélange de sang total et de tampon Hépès hépariné par le liquide de densité.

La présente invention a pour but de simplifier encore le procédé de séparation des basophiles selon les Brevets FR-A-2 460 481 et FR-A-2 483 621, tout en conservant les avantages et plus particulièrement l'avantage

2

représenté par la concentration élevée en polynucléaires basophiles du réactif et l'avantage représenté par le taux élevé de dégranulation observé dans le test de diagnostic des allergies et des parasitoses réalisé à l'aide du réactif.

Conformément à la présente invention, le procédé de préparation du réactif de diagnostic des parasitoses et des allergies consiste à déposer un échantillon de sang total prélevé de préférence en milieu d'acide éthylènediaminetétraacétique ou de l'un de ses sels, chez un patient humain ou animal supposé atteint d'une parasitose ou d'une allergie, mélangé à un volume égal du tampon Hépès hépariné décrit ci-dessus, sur un milieu de séparation des basophiles qui contient principalement du tampon Hépès, de la polyvinylpyrrolidone et un sel d'un acide iodobenzénique hydrosoluble, et dont la densité est de 1,077-1,085, puis à centrifuger cette composition pendant une durée et à une vitesse appropriées, pour obtenir un anneau contenant les basophiles et les lymphocytes, lequel est prélevé et lavé soigneusement avec du tampon Hépès de composition appropriée qui est ensuite rejeté pour ne conserver que le culot cellulaire qui, après remise en suspension, est prêt à être déposé dans les puits d'une lame ou d'une boîte de lecture de diagnostic dans certains desquels a été préalablement introduit du tampon et dans certains autres desquels ont été préalablement introduits des dilutions d'allergène(s) ou d'antigène(s) parasitaire(s).

Selon un mode de réalisation avantageux du milieu de séparation des basophiles mis en oeuvre conformément à la présente invention, celui-ci contient de préférence de l'héparine, de préférence sous la forme de l'un de ses sels alcalins et plus particulièrement de son sel de sodium.

Selon un autre mode de réalisation avantageux du milieu de séparation des basophiles mis en oeuvre conformément à la présente invention, le sel d'un acide iodobenzénique hydrosoluble qu'il comprend, est avantageusement pris dans le groupe qui comprend des sels d'acides triiodo- ou hexaiodobenzéniques hydrosolubles et plus particulièrement l'ioxitalamate de sodium et/ou de méglumine, l'ioxitalamate de méglumine et de monoéthanolamine et l'ioxaglate de sodium et/ou de méglumine.

Selon encore un autre mode de réalisation avantageux du milieu de séparation des basophiles mis en oeuvre conformément à la présente invention, celui-ci est constitué par une solution aqueuse contenant principalement de 3,5 à 55 ml/litre de tampon Hépès, de 5 à 30 g/litre de polyvinylpyrrolidone, de 220 à 250 ml/litre de sel d'acide iodobenzénique hydrosoluble.

Le milieu de séparation des basophiles conforme à la présente invention présente avantageusement la composition suivante:

Hépès, solution molaire 3,5 à 5,5 ml  
KCl 10 % 0,49 ml  
NaCl 9 % 45-48 ml  
Héparine sous la forme de l'un de ses sels alcalins 2500-7500 unités  
Polyvinylpyrrolidone 5-30 g  
Sel d'acide iodobenzénique hydrosoluble 220-250 ml  
Eau qsp 1 litre  
Densité 1,079-1,085  
pH 7,35-7,50 à 37° C

Conformément à la présente invention, le kit prêt à l'emploi pour la réalisation du test de diagnostic des parasitoses et des allergies comprend:

- un ensemble de composants pour la séparation des cellules basophiles, constitués par une pluralité de récipients appropriés tels qu'ampoules ou tubes contenant chacun une quantité prédosée d'un milieu de séparation des basophiles conforme à la présente invention, et une pluralité de récipients appropriés tels qu'ampoules ou tubes contenant chacun une quantité prédosée de tampon Hépès non destructeur à l'égard des cellules basophiles, pour le lavage de l'anneau lymphocytaire séparé par le milieu de séparation; - et un ensemble de composants pour la réalisation du test de diagnostic, constitués par une pluralité de lames ou de boîtes de lecture de diagnostic, présentant chacune un certain nombre de puits dont la majeure partie, à l'exception des puits témoins, sont destinés à recevoir des quantités prédéterminées variables d'un ou de plusieurs antigènes spécifiques d'une ou de plusieurs allergies ou d'une ou plusieurs parasitoses à diagnostiquer ou à dépister, - une pluralité d'ampoules ou de tubes renfermant des quantités prédosées d'une solution de fixation et de coloration rapides, simultanées, du type des solutions telles que définies dans le Brevet FR-A-2 483 621, un tube de milieu de montage optique destiné à être disposé entre les lames de lecture préparées pour le diagnostic et des lamelles de protection, - et une pluralité de lamelles de protection.

Grâce à la mise au point d'un milieu de séparation approprié, c'est le sang prélevé (avantageusement sur du sel de potassium de l'acide éthylènediaminetétraacétique) préalablement mélangé avec du tampon Hépès, qui est directement déposé, sans centrifugation préalable, sur le milieu de densité qui réalise la séparation lors d'une centrifugation postérieure, ce processus simplifié étant rendu possible par la présence dans le milieu de densité, d'un composé iodé qui favorise la séparation de l'anneau contenant les basophiles et les lymphocytes.

Outre les dispositions qui précèdent, l'invention comprend encore d'autres dispositions qui ressortiront de la description qui va suivre.

L'invention sera mieux comprise à l'aide du complement de description qui va suivre, qui se réfère à un exemple de mise en oeuvre du procédé objet de la présente invention.

Il doit être bien entendu, toutefois, que cet exemple de mise en oeuvre est donné uniquement à titre d'illustration de l'objet de l'invention, dont il ne constitue en aucune manière une limitation.

**EKEMPLE**

I- Préparation d'un réactif de diagnostic de parasitoses et d'allergies

1° - On prélève chez un patient à jeûn, supposé atteint d'une ou de plusieurs parasitoses ou allergies, 5 ml de sang veineux sur de l'EDTA K$_3$ pour avoir une concentration finale de 1,5 mg d'EDTA K$_3$ par ml de sang; on le mélange à 5 ml de tampon Hépès contenant de l'héparine.

2° - Dans un tube (1) à fond rond centrifugeable, on place 5 ml d'un milieu de séparation de basophiles dont la composition est la suivante:

Hépès, solution molaire 0,369 ml

KCl 10 % 0,049 ml

NaCl 9 % 4,63 ml

Héparinate de sodium 263 unités (1,65 mg)

Polyvinylpyrrolidone 2 g

Ioxaglate de sodium et de méglumine (diffusé sous la marque "HEXABRIX" des Laboratoires GUERBET) 23,7 ml

H$_2$O qsp 95 ml + 5 ml environ pour ajuster la densité à 1,080 g/ml

pH 7,42 à 37°C

3° - On dépose doucement dans ce tube (1) à la surface du milieu de séparation, à l'aide d'une pipette Pasteur, le mélange volume à volume d'échantillon de sang et de tampon Hépès contenant de l'héparine, préparé en 1°-ci-dessus.

4° - On centrifuge ce tube pendant 30 minutes, à 400 g. Le tampon Hépès contenant le KCl et le NaCl se sépare du milieu de séparation proprement dit constitué par le mélange de l'"HEXABRIX" et de polyvinylpyrrolidone dans un tampon Hépès, et à la limite entre les deux, il se forme un anneau blanchâtre que l'on prélève en totalité (1 à 3 ml) à l'aide d'une pipette Pasteur montée sur seringue ou poire.

5° - Le milieu cellulaire anisi obtenu est déposé dans un tube (2) à centrifuger contenant du milieu tampon Hépès hépariné présentant la composition suivante:

Hépès, solution molaire 4 à 25 ml

KCl 10 % 0,932 ml

NaCl 9 % 85 à 90 ml

Héparinate de sodium 5000 à 10000 unités

Eau qsp 1000 ml

pH 7,4-7,6 à 37°C

à l'aide duquel il est lavé au cours d'une centrifugation de 10 minutes à 150 g. Le surnageant est prélevé jusqu'à la rupture de pente du tube (volume restant ± 0,5 ml), ou bien on peut, en variante, de façon tout aussi efficace, éliminer le surnageant en retournant le tube 3 secondes et reprendre le culot cullulaire par environ 0,5 ml du même tampon Hépès.

Dans les deux cas, le culot cellulaire est homogénéisé totalement pendant 30-50 secondes par agitation manuelle ou au VORTEX (Marque déposée).

II - Test de diagnostic

1°- Le culot cellulaire mis en suspension est déposé par fractions de 20 µl dans chacun des puits d'une lame de lecture de diagnostic, qui peuvent éventuellement contenir du CaCl$_2$ et du MgCl$_2$. La lame est ensuite mise dans une boîte couverte (type boâ6te de Pétri) contenant un morceau de coton humide. L'ensemble est placé à l'étuve à 37°C pendant 20 minutes.

Puis le couvercle de la boîte et le coton sont enlevés. La lame, toujours à l'étuve, est ainsi séchée en environ 15 à 30 minutes. On peut aussi sécher les lames au sèche-cheveux.

La fixation/coloration est obtenue en recouvrant chacun des puits d'une solution de coloration et de fixation rapides, simultanées, décrite dans l'Art antérieur. La lame est ainsi laissée sur la paillasse pendant 15 minutes, puis est rincée dans un bain d'eau (30 secondes), déshydratée dans un bain d'éthanol absolu (20 à 30 secondes) et plongée quelques secondes dans un bain de xylène pour faciliter le montage.

Une goutte de milieu de montage est déposée sur une lamelle. Celle-ci est ensuite appliquée sur la lame. 10 à 15 minutes sont nécessaires pour sécher l'ensemble (on peut accélérer le séchage en mettant la lame à l'étuve ou sous un sèche-cheveux). ·

2° - Lecture du test

Elle est réalisée au microscope optique ordinaire. Il est préférable de disposer d'objectifs et d'oculaires "grand-champ" et de qualité suffisante pour procurer une mise au point parfaite sur l'ensemble du champ. Elle peut avantageusement être pratiquée à l'aide d'une caméra de télévision montée sur le microscope et reliée à un "ordinateur" type "OPTOMAX" (Marque déposée) en noir et blanc. Cette technique de lecture du test est décrite dans "ALLERGY", 1981, 36, 239-244, F. LEYNADIER, H. LUCE, A. ABREGO et J. DRY "Automated measurement of human basophil degranulation".

Le grandissement utilisé est variable avec le nnombre de basophiles. En général de 400, il peut être porté à 1000 (immersion) en cas de grande richesse en basophiles, ou au contraire à 250 si l'observateur est habitué.

Les basophiles sont reconnus grâce a leur coloration foncée par rapport aux autres cellules (essentiellement lymphocytes et monocytes) et surtout à la teinte violette parfois rouge différente de la coloration bleue (claire ou foncée) des autres cellules.

Les basophiles sont comptés sur chacun des puits. Dans la grande majorité des cas, vingt champs

4

microscopiques sont suffisants (dix champs au hasard dans un diamètre vertical et dix champs au hasard dans un diamètre horizontal). L'important est, bien entendu, qu'un même nombre de champs soit examiné sur chacun des puits.

En ajoutant les nombres de basophilrs trouvés dans les puits identiques A et B, on obtient:

T = nombre de basophiles sans antigène ou nombre total de basophiles

Ag (1,2, 3 ou 4) = nombre de basophiles avec antigène(s) à la dilution 1, 2, 3 ou 4

On détermine l'index de dégranulation (I.D.) pour chaque dilution d'antigène(s) par la relation suivante:

$$I.D.\% = \frac{T-Ag}{T} \times 100$$

c'est-à-dire le nombre de basophiles ayant dégranulé a une dilution donnée par rapport au nombre total de basophiles présents dans le témoin T (cette valeur étant exprimée en pourcentage).

Le Tableau ci-dessous montre que le pourcentage de dégranulation est sensiblement du même ordre lorsqu'il est pratiqué avec du réactif constitué par des basophiles isolés de sujets allergiques en utilisant le procédé de préparation du réactif qui fait l'objet du Brevet FR-A-2 483 621, et avec du réactif obtenu par le procédé qui fait l'objet de la présente invention.

**Tableau**

| Nom du malade | Date | Test,exprimé en % de dégranulation, effectué avec du réactif constitué par des basophiles isolés à l'aide du | | | |
| | | milieu de séparation selon le Brevet FR-A-2 483 621 | | milieu de séparation selon la présente invention contenant 2 % de PVP | |
| | | T | % | T | % |
| PL. | 3/12/81 | 50 | 0 | | |
| Gwenolla L. | 3/12/81 | 146 | | | |
| Francis C. | 3/12/81 | 125 | 88 | | |
| Nicole P. | 3/12/81 | 129 | 0 | | |
| J.Ph. S. | 7/12/81 | | | 150 | 0 |
| Jean G. | 7/12/81 | | | 129 | 98 |
| Martine M. | 7/12/81 | | | 106 | 0 |
| Gilles G. | 9/12/81 | | | 158 | 93 |
| Eliane G. | 10/12/81 | 148 | 42 | 116 | 64 |
| Madeleine L. | 10/12/81 | | | 116 | 72 |
| Christian P. | 10/12/81 | 84 | 0 | 151 | 9 |
| Yvonne B. | 10/12/81 | 80 | 0 | | |
| Ibrahima D. | 29/12/81 | 93 | 32 | | |
| Emile S. | 5/01/82 | 158 | 0 | 141 | 0 |
| Claude B. | 5/01/82 | 139 | 92 | 137 | 89 |
| Michel O. | 5/01/82 | 153 | 41 | 154 | 36 |
| Pascal B. | 7/01/82 | 118 | 0 | 132 | 0 |
| Lev. | 25/01/82 | 115 | | 154 | |
| Dupont | 25/01/82 | 175 | | 115 | |
| Gesl. | 25/01/82 | 100 | | 154 | |
| Palv. | 25/01/82 | 67 | | 111 | |
| Ren. | 27/01/82 | 93 | | 100 | |

T = Nombre de basophiles comptés sur 10 champs microscopiques en l'absence d'antigène (témoin).

Le réactif de diagnostic conforme aux dispositions de la présente invention est utilisable non seulement pour la réalisation de tests de diagnostic ou de dépistage de parasitoses et d'allergies, mais également au diagnostic d'autres maladies et notamment d'affections bactériennes, immunopathologiques, virales et mycologiques, en particulier, qui provoquent une augmentation des immunoglobulines spécifiques circulantes, et notamment des IgE.

**Revendications** pour les Etats contractants BC, CH, DE, BG, LI, LU, NL, SE

1°) Procédé de préparation d'un réactif de diagnostic des parasitoses et des allergies qui provoquent une augmentation des immunoglobulines circulantes et notamment de IgE lequel réactif de diagnostic est constitué par une suspension de polynucléaires basophiles porteurs d'IgE spécifiques contenant de 300 à 1 000 basophiles par mm$^3$, obtenue à partir d'un échantillon de sang prélevé chez un patient chez lequel on recherche une parasitose ou une allergie par centrifugation pour recueillir une couche contenant essentiellement des leucocytes, qui est mélangée avec un tampon non destructeur vis-à-vis des cellules basophiles, tel que le tampon Hépès (acide N-2-hydroxyéthylpipérazine-N'-2-éthanesulfonique) 1M ou le tampon Pipès (acide pipérazine-N,N'-bis-2-éthanesulfonique) notamment, ledit mélange homogénéisé étant ensuite déposé sur un liquide de densité 1,079-1,085 dont l'osmolarité est comprise enüre 280 et 320 milliosmoles, pour donner lieu, après centrifugation pendant une durée et à une vitesse appropriées, à un anneau qui est constitué par une suspension enrichie en basophiles et qui surmonte le liquide de densité, lequel anneau est prélevé, puis lavé, de préférence à l'aide du même tampon que ci-dessus, pour être déposé dans les différents puits d'une lame ou boîte de lecture de diagnostic, ledit procédé étant caractérisé en ce qu'au cours d'une première étape, un échantillon de sang total, prélablement prélevé de préférence en milieu d'acide éthylènediaminetétraacétique à l'état libre ou sous forme de l'un de ses sels, chez un patient humain ou animal supposé atteint d'une ou de plusieurs parasitoses ou d'une ou de plusieurs allergies, mélangé sensiblement volume à volume avec un tampon Hépès, est déposé sur un milieu de séparation des basophiles qui contient principalement du tampon Hépès, de la polyvinylpyrrolidone et un sel d'un acide iodobenzénique hydrosoluble, et dont la densité est de 1,077-1,085, puis en ce qu'au cours d'une deuxième étape, cette composition est centrifugée pendant une durée et à une vitesse appropriées, pour obtenir un anneau contenant les basophiles et les lymphocytes, lequel est prélevé et lavé soigneusement avec du tampon Hépès de composition appropriée qui est ensuite rejeté pour ne conserver que le culot cellulaire qui, après remise en suspension, est prêt à être déposé dans les puits d'une lame ou d'une boîte de lecture de diagnostic dans certains desquels a été préalablement introduit du tampon et dans certains autres desquels ont été préalablement introduites des dilutions d'allergène(s) ou d'antigène(s) parasitaire(s).

2° - Procédé selon la Revendication 1, caractérisé en ce que le milieu de séparation des basophiles mis en oeuvre au cours de la première étape du procédé contient de préférence de l'héparine, de préférence sous la forme de l'un de ses sels alcalins, et plus particulièrement de son sel de sodium.

3°- Procédé selon la Revendication 1, caractérise en ce que le sel d'un acide iodobenzénique hydrosoluble que contient le milieu de séparation susdit, est avantageusement pris dans le groupe qui comprend des sels d'acides triiodo- ou hexaiodobenzéniques hydrosolubles et plus particulièrement l'ioxitalamate de sodium et/ou de méglumine, l'ioxitalamate de méglumine et de monoéthanolamine, l'ioxaglate de sodium et/ou de méglumine.

4°- Procédé selon la Revendication 1, caractérisé en ce que ledit milieu de séparation des basophiles est constitué par une solution contenant principalement de 3,5 à 5,5 ml/litre de tampon Hépès, de 5 à 30 g/litre de polyvinylpyrrolidone, de 220 à 250 ml/litre de sel d'acide iodobenzénique hydrosoluble.

5°- Procédé selon la Revendication 1, caractérisé en ce que ledit milieu de séparation des basophiles présente avantageusement la composition suivante:

Hépès, solution molaire 3,5 à 5,5 ml

KCl 10 % 0,49 ml

NaCl 9 % 45-48 ml

Héparine sous la forme de l'un de ses sels alcalins 2500-7500 unités

Polyvinylpyrrolidone 5-30 g

Sel d'acide iodobenzénique hydrosoluble 220-250 ml

Eau qsp 1 litre

Densité 1,079-1,085

pH 7,35-7,50 à 37°C.

6°- Réactif de diagnostic d'affections qui provoquent une augmentation des immunoglobulines spécifiques circulantes, et notamment des IgE, caractérisé en ce qu'il est constitué par une suspension de globules blancs porteurs d'IgE spécifiques, obtenue à partir d'un échantillon de sang prélevé chez un patient humain ou animal devant être soumis à un test de diagnostic, laquelle suspension est enrichie en lesdits globules blancs porteurs d'IgE spécifiques (basophiles) et est obtenue par centrifugation d'une composition constituée par un mélange sensiblement volume à volume dudit échantillon de sang et d'un tampon Hépès de préférence hépariné, déposé sur un milieu de séparation desdits globules blancs, comprenant essentiellement du tampon Hépès, de la polyvinylpyrrolidone et un sel d'un acide iodobenzénique hydrosoluble, et dont la densité est de 1,077-1,085, le culot cellulaire résultant de ladite centrifugation étant constitué par la suspension de globules blancs porteurs d'IgE spécifiques (basophiles) qui forme le réactif de diagnostic.

7° - Milieu de séparation pour la préparation du réactif de diagnostic selon la Revendication 5 ci-dessus, caractérisé en ce qu'il contient principalement du tampon Hépès, de la polyvinylpyrrolidone et un sel d'un acide iodobenzénique hydrosoluble, et dont la densité est de 1,077-1,085.

8° - Milleu de séparation selon la Revendication 7 ci-dessus, caractérisé en ce qu'il est constitué par une solution contenant principalement de 3,5 à 5,5 ml/litre de tampon Hépès, de 5 à 30 g/litre de polyvinylpyrrolidone, de 220 à 250 ml/litre d'un ou plusieurs sels d'acides iodobenzéniques hydrosolubles.

9°- Milieu de séparation selon la Revendication 8 ci-dessus, caractérisé en ce qu'il présente avantageusement la composition suivante:

Hépès, solution molaire 3,5 à 5,5 ml
KCl 10 % 0,49 ml
NaCl 9 % 45-48 ml
Héparine sous la forme de l'un de ses sels alcalins 2500-7500 unités
Polyvinylpyrrolidone 5-30 g
Sel d'acide iodobenzénique hydrosoluble 220-250 ml
Eau qsp 1 litre
Densité 1,079-1,085
pH 7,35-7,50 à 37°C

10°- Kits prêts à l'emploi pour la réalisation du test de diagnostic des parasitoses et des allergies caractérisés en ce qu'ils comprennent:

- un ensemble de composants pour la séparation des cellules basophiles, constitués par une pluralité de récipients appropriés tels qu'ampoules ou tubes contenantchacunune quantité prédosée du milieu de séparation des basophiles selon la Revendication 5 ci-dessus, et une pluralité de récipients appropriés tels qu'ampoules ou tubes contenant chacun une quantité prédosée d'un tampon Hépès de composition appropriée pour le lavage de l'anneau lymphocytaire séparé séparé par le milieu de séparation, lequel tampon Hépès présente avantageusement la composition suivante connue en elle-même:

Tampon Hépès, solution molaire 4 à 25 ml
KCl 10 % 0,932 ml
CaCl$_2$ 10 % 0,550 ml
MgCl$_2$ 10 % 0,510 ml
NaCl 9 % 850 è 900 ml
Héparine 10 unités/ml
Eau q.s.p. 1000 ml
pH 7,4 - 7,6

- et un ensemble de composants pour la réalisation du test de diagnostic, constitués par une pluralité de lames ou de boîtes de lecture de diagnostic, présentant chacune un certain nombre de puits dont la majeure partie, à l'exception des puits témoins, sont destinés à recevoir des quantités prédéterminées variables d'un ou plusieurs antigènes spécifiques d'une ou plusieurs allergies ou d'une ou plusieurs parasitoses à diagnostiquer ou à dépister, - une pluralité d'ampoules ou de tubes renfermant des quantités prédosées d'une solution de fixation et de coloration rapides, simultanées, - un tube de milieu de montage optique, - et une pluralité de lamelles de protection.

11° - Application du réactif de diagnostic selon la Revendication 5 ci-dessus au diagnostic d'une parasitose ou d'une allergie, au diagnostic simultané de plusieurs parasitoses et/ou allergies et au diagnostic d'autres affections qui provoquent une augmentation des immunoglobulines spécifiques circulantes et notamment des IgE, telles que les affections bactériennes, immunopathologiques, virales et mycologiques, en particulier.


**Revendications** pour l'Etat contractant AT

1°) Procédé de préparation d'un réactif de diagnostic des parasitoses et des allergies qui provoquent une augmentation des immunoglobulines circulantes et notamment de, IgE lequel réactif de diagnostic est constitué par une suspension de polynucléaires basophiles porteurs d'IgE spécifiques contenant de 300 à 1 000 basophiles par mm$^3$, obtenue à partir d'un échantillon de sang prélevé chez un patient chez lequel on recherche une parasitose ou une allergie par centrifugation pour recueillir une couche contenant essentiellement des leucocytes, qui est mélangée avec un tampon non destructeur vis-à-vis des cellules basophiles, tel que le tampon Hépès (acide N-2 hydroxyéthyl-pipérazine-N'-2-éthanesulfonique) 1M ou le tampon Pipès (acide pipérazine-N,N'-bis-2-éthanesulfonique) notamment, ledit mélange homogénéisé étant ensuite déposé sur un liquide de densité 1,079-1,085 dont l'osmolarité est comprise entre 280 et 320 milliosmoles, pour donner lieu, après centrifugation pendant une durée et à une vitesse appropriées, à un anneau qui est constitué par une suspension enrichie en basophiles et qui surmonte le liquide de densité, lequel anneau est prélevé, puis lavé, de préférence à l'aide du même tampon que ci-dessus, pour être déposé dans les différents puits d'une lame ou boîte de lecture de diagnostic, ledit procédé étant caractérisé en ce qu'au cours d'une première étape, un échantillon de sang total, préalablement prélevé de préférence en milieu d'acide éthylènediaminetétraacétique à l'état libre ou sous forme de l'un de ses sels, chez un patient humain ou animal supposé atteint d'une ou de plusieurs parasitoses ou d'une ou de plusieurs allergies, mélangé sensiblement volume à volume avec un tampon Hépès, est déposé sur un milieu de séparation des basophiles qui contient principalement du tampon Hépès, de la polyvinylpyrrolidone et un sel d'un acide iodobenzénique hydrosoluble, et dont la densité est de 1,077-1,085, puis en ce qu'au cours d'une deuxième étape, cette composition est centrifugée pendant une durée et à une vitesse appropriées, pour obtenir un anneau contenant les basophiles et les lymphocytes, lequelest prélevé et lavé soigneusement avec du tampon Hépès de

8

composition appropriée qui est ensuite rejeté pour ne conserver que le culot cellulaire qui, après remise en suspension, est prêt à être déposé dans les puits d'une lame ou d'une boîte de lecture de diagnostic dans certains desquels a été préalablement introduit du tampon et dans certains autres desquels ont été préalablement introduites des dilutions d'allergène(s) ou d'antigène(s) parasitaire(s).

2° - Procédé selon la Revendication 1, caractérisé en ce que le milieu de séparation des basophiles mis en oeuvre au cours de la première étape du procédé contient de préférence de l'héparine, de préférence sous la forme de l'un de ses sels alcalins, et plus particulièrement de son sel de sodium.

3° - Procédé selon la Revendication 1, caractérisé en ca que le sel d'un acide iodobenzénique hydrosoluble que contient le milieu de séparation susdit, est avantageusement pris dans le groupe qui comprend des sels d'acides triiodo- ou hexaiodobenzéniques hydrosolubles et plus particuliérement l'ioxitalamate de sodium et/ou de méglumine, l'ioxitalamate de méglumine et de monoéthanolamine, l'ioxaglate de sodium et/ou de méglumine.

4° - Procédé selon la Revendication 1, caractérisé en ce que ledit milieu de séparation des basophiles est constitué par une solution contenant principalement de 3,5 à 5,5 ml/litre de tampon Hépès, de 5 à 30 g/litre de polyvinylpyrrolidone, de 220 à 250 ml/litre de sel d'acide iodobenzénique hydrosoluble.

5° - Procédé selon la Revendication 1, caractérisé en ce que ledit milieu de séparation des basophiles présente avantageusement la composition suivante:

Hépès, solution molaire 3,5 à 5,5 ml
KCl 10 % 0,49 ml
NaCl 9 % 45-48 ml
Héparine sous la forme de l'un de ses sels alcalins 2500-7500 unités
Polyvinylpyrrolidone 5-30 g
Sel d'acide iodobenzénique hydrosoluble 220-250 ml
Eau qsp 1 litre
Densité 1,079-1,085
pH 7,35-7,50 à 37°C.

6° - Procédé pour la préparation d'un réactif de diagnostic d'affections qui provoquent une augmentation des immunoglobulines spécifiques circulantes, et notamment des IgE, caractérisé en ce qu'il est constitué par une suspension de globules blancs porteurs d'IgE spécifiques, obtenue à partir d'un échantillon de sang prélevé chez un patient humain ou animal devant être soumis à un test de diagnostic, laquelle suspension est enrichie en lesdits globules blancs porteurs d'IgE spécifiques (basophiles) et est obtenue par centrifugation d'une composition constituée par un mélange sensiblement volume à volume dudit échantillon de sang et d'un tampon Hépès de préférence hépariné, déposé sur un milieu de séparation desdits globules blancs, comprenant essentiellement du tampon Hépès, de la polyvinylpyrrolidone et un sel d'un acide iodobenzénique hydrosoluble, et dont la densité est de 1,077-1,085, le culot cellulaire résultant de ladite centrifugation étant constitué par la suspension de globules blancs porteurs d'IgE spécifiques (basophiles) qui forme le réactif de diagnostic.

7° - Procédé de préparation d'un milieu de séparation pour la préparation du réactif de diagnostic selon la revendication 5 ci-dessus, caractérisé en ce qu'il contient principalement du tampon Hépès, de la polyvinylpyrrolidone et un sel d'un acide iodobenzénique hydrosoluble, et dont la densité est de 1,077-1,085.

8° - Procédé de préparation d'un milieu de séparation selon la revendication 7 ci-dessus, caractérisé en ce qu'il est constitué par une solution contenant principalement de 3,5 à 5,5 ml/litre de tampon Hépès, de 5 à 30g/litre de polyvinylpyrrolidone, de 220 à 250 ml/litre d'un ou plusieurs sels d'acides iodobenzéniques hydrosolubles.

9° - Procédé de préparation d'un milieu de séparation selon la revendication ci-dessus, caractérisé en ce qu'il présente avantageusement la composition suivante:

Hépès, solution molaire 3,5 à 5,5 ml
KCl 10 % 0,49 ml
NaCl 9 % 45-48 ml
Héparine sous la forme de l'un de ses sels alcalins 2500-7500 unités
Polyvinylpyrrolidone 5-30 g
Sel d'acide iodobenzénique hydrosoluble 220-250 ml
Eau qsp 1 litre
Densité 1,079-1,085
pH 7,35-7,50 à 37°C

10° - Procédé de préparation de kits prêts à l'emploi pour la réalisation du test de diagnostic des parasitoses et des allergies caractérisés en ce qu'ils comprennent:

- un ensemble de composants pour la séparation des cellules basophiles, constitués par une pluralité de récipients appropriés tels qu'ampoules ou tubes contenant chacun une quantité prédosée du milieu de séparation des basophiles selon la Revendication 5 ci-dessus, et une pluralité de récipients appropriés tels qu'ampoules ou tubes contenant chacun une quantité prédosée d'un tampon Hépès de composition appropriée pour le lavage de l'anneau lymphocytaire séparé séparé par le milieu de séparation, lequel tampon Hépès présente avantageusement la composition suivante connue en elle-même:

Tampon Hépès, solution molaire 4 à 25 ml
KCl 10 % 0,932 ml

9

CaCl$_2$ 10 % 0,550 ml
MgCl$_2$ 10 % 0,510 m1
NaCl 9 % 850 à 900 ml
Héparine 10 unités/ml
Eau q.s.p. 1000 ml
pH 7,4 - 7,6

- et un ensemble de composants pour la réalisation du test de diagnostic, constitués par une pluralité de lames ou de boîtes de lecture de diagnostic, présentant chacune un certain nombre de puits dont la majeure partie, à l'exception des puits témoins, sont destinés à recevoir des quantités prédéterminées variables d'un ou plusieurs antigènes spécifiques d'une ou plusieurs allergies ou d'une ou plusieurs parasitoses à diagnostiquer ou à dépister, - une pluralité d'ampoules ou de tubes renfermant des quantités prédosées d'une solution de fixation et de coloration rapides, simultanées, - un tube de milieu de montage optique, - et une pluralité de lamelles de protection.

11°- Application du réactif de diagnostic selon la Revendication 5 ci-dessus au diagnostic d'une parasitose ou d'une allergie, au diagnostic simultané de plusieurs parasitoses et/ou allergies et au diagnostic d'autres affections qui provoquent une augmentation des immunoglobulines spécifiques circulantes et notamment des IgE, telles que les affections bactériennes, immunopathologiques, virales et mycologiques, en particulier.

**Patentansprüche** für die Vertragsstaaten BE, CH, DE, BG, LI, LU, NL, SE,

1. Verfahren zur Herstellung eines Reagenz zur Diagnose von Parasitosen und Allergien, die eine Vermehrung der zirkulierenden Immunglobuline und insbesondere der IgE hervorrufen, wobei das Reagenz zur Diagnose durch eine Suspension polynuklearer basophiler Träger spezifischer IgE mit einem Gehalt von 300 bis 1000 Basophile pro mm$^3$ gebildet wird, erhaltend ausgehend von einer Blutprobe, die einem Patienten entnommen wurde, bei dem man eine Parasitose oder eine Allergie festzustellen versucht, durch Zentrifugieren, um eine Schicht zu erhalten, die im wesentlichen Leukozyten enthält, die mit einem Puffer vermischt wird, der nicht zerstörend auf die basophilen Zellen wirkt, wie insbesondere Hepes-Puffer (N-2-hydroxyethylpiperazin-N'-2-ethansulfonsäure), 1M, oder Pipes-Puffer (Piperazin-N,N'-bis-2-ethansul-fonsäure), worauf das homogenisierte Gemisch auf einer Flüssigkeit mit einer Dichte von 1,079-1,085, deren Osmolarität bei 280 bis 320 Milliosmol liegt, abgelagert wird, zur Bildung, nach dem Zentrifugieren während geeigneter Dauer und bei geeigneter Geschwindigkeit, von einem Ring, der aus einer Suspension besteht, die mit Basophilen angereichert ist, und der eine größere Dichte als die Flüssigkeit aufweist, worauf dieser Ring entnommen und dann vorzugsweise mit dem gleichen Puffer wie vorstehend gewaschen wird, zur Abscheidung in verschiedenen Vertiefungen einer Platte oder eines Kastens zur diagnostischen Ablesung, wobei dieses Verfahren <u>dadurch gekennzeichnet</u> ist, daß man im Verlauf einer ersten Stufe eine Probe des Gesamtblutes, die vorausgehend vorzugsweise in einem Medium von Ethylendiamintetraessigsäure im freien Zustand oder in der Form eines ihrer Salze von einem menschlichen Patienten oder von einem Tier entnommen wurde, von dem angenommen wird, daß er bzw. es von einer oder mehreren Parasitosen oder einer oder mehreren Allergien befallen wurde, die genau Volumen zu Volumen mit einem Hepes-Puffer vermischt wurde, auf einem Medium zur Abtrennung der Basophile abgelagert wird, das hauptsächlich Hepes-Puffer, Polyvinylpyrrolidon und ein wasserlösliches Salz einer Jodbenzolsäure enthält, und dessen Dichte 1,077-1,085 beträgt, das anschließend im Verlauf einer zweiten Stufe diese Zusammensetzung bei geeigneter Dauer und Geschwindigkeit zentrifugiert wird, unter Erzielung eines Ringes, der die Basophile und die Lymphozyten enthält, der entnommen und sorgfältig mit Hepes-Puffer gewaschen wird, der eine geeignete Zusammensetzung aufweist und anschließend verworfen wird, wobei nur der Zellbodensatz behalten wird, der nach der erneuten Suspendierung geeignet ist zur Ablagerung in Vertiefungen einer Platte oder eines Kastens zur diagnostischen Ablesung, wobei in bestimmte davon vorher ein Puffer eingeführt wurde und in bestimmte andere davon vorher Verdünnungen eines oder mehrerer parasitärer Allergene oder Antigene eingebracht wurden.

2. Verfahren nach Anspruch 1, dadurch gekennzeichnet, daß das Trennmedium für die Basophile, das im Verlauf der ersten Stufe des Verfahrens eingesetzt wird, vorzugsweise Heparin, bevorzugt in der Form eines seiner Alkalisalze und besonders bevorzugt seines Natriumsalzes, enthält.

3. Verfahren nach Anspruch 1, dadurch gekennzeichnet, daß das wasserlösliche Salz einer Jodbenzolsäure, das in dem vorstehenden Trennmedium enthalten ist, vorteilhaft aus der Gruppe genommen wird, die wasserlösliche Salze von Trijod- oder Hexajodbenzolsäuren und insbesondere Natrium- und/oder Megluminioxitalamat, Meglumin- und Monoethanolaminioxitalamat, Natrium- und/oder Meglumin-ioxaglat, umfaßt.

4. Verfahren nach Anspruch 1, dadurch gekennzeichnet, daß das Trennmedium für die Basophile aus einer Lösung besteht, die hauptsächlich 3,5 bis 5,5 ml/l Hepes-Puffer, 5 bis 30 g/l Polyvinylpyrrolidon, 220 bis 250 ml/l wasserlösliches Jodbenzolsäuresalz enthält.

5. Verfahren nach Anspruch 1, dadurch gekennzeichnet, daß das Trennmedium für die Basophile vorteilhaft folgende Zusammensetzung aufweist:
Hepes, molare Lösung 3,5 bis 5,5 ml
KCl 10 % 0,49 ml

**0 099 812**

NaCl 9 % 45 bis 48 ml
Heparin in der Form eines seiner Alkalisalze 2500 - 7500 Einheiten
Polyvinylpyrrolidon 5 - 30 g
wasserlösliches Jodbenzolsäuresalz 220 - 250 ml
Wasser qsp 1 Liter
Dichte 1,079 - 1,085
pH 7,35 - 7,50 bei 37° C

6. Reagenz zur Diagnose von Erkrankungen, die eine Vermehrung spezifischer zirkulierender Immunglobuline und insbesondere von IgE hervorrufen, dadurch gekennzeichnet, daß es aus einer Suspension weißer Blutkörperchen, die spezifische IgE tragen, erhalten ausgehend von einer Blutprobe, entnommen von einem menschlichen Patienten oder einem Tier, der bzw. das einem diagnostischen Test unterzogen werden soll, enthält, wobei die Suspension an diesen weißen Blutkörperchen, die spezifische IgE (Basophile) tragen, angereichert ist und erhalten wurde durch Zentrifugieren einer Zusammensetzung, die aus einem Gemisch von genau Volumen zu Volumen der Blutprobe und einem Hepes-Puffer, vorzugsweise heparinisiert, abgelagert auf einem Trennmedium für die weißen Blutkörperchen, das im wesentlichen Hepes-Puffer, Polyvinylpyrrolidon und ein wasserlösliches Salz einer Jodbenzolsäure enthält, und dessen Dichte 1,077-1,085 beträgt, wobei der Zell-Bodensatz der Zentrifugation aus einer Suspension von weißen Blutkörperchen besteht, die spezifische IgE (Basophile) tragen, die das Reagenz zur Diagnose bildet.

7. Trennmedium zur Herstellung des diagnostischen Reagenz nach dem vorstehenden Anspruch 5, dadurch gekennzeichnet, daß es hauptsächlich Hepes-Puffer, Polyvinylpyrrolidon und ein wasserlösliches Salz einer Jodbenzolsäure enthält und dessen Dichte 1,077-1,085 beträgt.

8. Trennmedium nach dem vorstehenden Anspruch 7, dadurch gekennzeichnet, daß es aus einer Lösung besteht, die hauptsächlich 3,5 bis 5,5 ml/l Hepes-Puffer, 5 bis 30 g/l Polyvinylpyrrolidon, 220 bis 250 ml/l eines oder mehrerer wasserlöslicher Salze von Jodbenzolsäuren enthält.

9. Trennmedium nach dem vorstehenden Anspruch 8, dadurch gekennzeichnet, daß es vorteilhaft die folgende Zusammensetzung aufweist:
Hepes, molare Lösung 3,5 bis 5,5 ml
KCl 10 % 0,49 ml
NaCl 9 % 45 - 48 ml
Heparin in der Form eines seiner Alkalisalze 2500 - 7500 Einheite
Polyvinylpyrrolidon 5 - 30 g
wasserlösliches Jodbenzolsäuresalz 220 - 250 ml
Wasser qsp 1 Liter
Dichte 1,079 - 1,085
pH 7,35 - 7,50 bei 37° C

10. Kits, bereit zur Verwendung zur Durchführung des diagnostischen Tests von Parasitosen und Allergien, dadurch gekennzeichnet, daß sie umfassen:
- die Gesamtheit der Bestandteile zur Trennung der basophilen Zellen, bestehend aus mehreren geeigneten Rezipienten, wie Ampullen oder Röhrchen, die jeweils eine vordosierte Menge des Trennmediums für die Basophile gemäß dem vorstehenden Anspruch 5 enthalten, sowie mehrere geeignete Rezipienten, wie Ampullen oder Röhrchen, die jeweils eine vordosierte Menge eines Hepes-Puffers mit geeigneter Zusammensetzung zur Wäsche des abgetrennten Lymphozytenringes, abgetrennt durch das Trennmedium, enthalten, wobei der Hepes-Puffer vorteilhaft folgende an sich bekannte Zusammensetzung aufweist:
Hepes-Puffer, molare Lösung 4 - 25 ml
KCl 10 % 0,932 ml
CaCl$_2$ 10 % 0,550 ml
MgCl$_2$ 10 % 0,510 ml
NaCl 9 ‰ 850 - 900 ml
Heparin 10 Einheiten/ml
Wasser q.s.p. 1000 ml
pH 7,4 - 7,6
- und die gesamten Bestandteile zur Durchführung des diagnostischen Tests, bestehend aus mehreren Platten oder Kästen zur diagnostischen Ablesung, die jeweils eine bestimmte Anzahl von Vertiefungen aufweisen, deren Hauptteil, mit Ausnahme der Kontrollvertiefungen, bestimmt ist zur Aufnahme vorbestimmter variabler Mengen eines oder mehrerer Antigene, die spezifisch für eine odere mehrere Allergien oder eine oder mehrere Parasitosen sind, die diagnostisch bestimmt oder aufgespürt werden sollen,
- mehrere Ampullen oder Röhrchen, die vordosierte Mengen einer Lösung zur raschen gleichzeitigen Fixierung und Färbung enthalten,
- ein Röhrchen mit optischem Befestigungsmedium,
- und mehrere Schutzlamellen.

11. Verwendung des diagnostischen Reagenz nach dem vorstehenden Anspruch 5 zur Diagnose einer Parasitose oder einer Allergie zur gleichzeitigen Diagnose verschiedener Parasitosen und/oder Allergien und zur Diagnose anderer Erkrankungen, die eine Vermehrung der spezifischen zirkulierenden Immunglobuline und insbesondere der IgE hervorrufen, wie insbesondere bakterielle, immunpathologische, virale und mykologische Erkrankungen.

**Patentansprüche für den Vertragsstaat AT**

1. Verfahren zur Herstellung eines Reagenz zur Diagnose von Parasitosen und Allergien, die eine Vermehrung der zirkulierenden Immunglobuline und insbesondere der IgE hervorrufen, wobei das Reagenz zur Diagnose durch eine Suspension polynuklearer basophiler Träger spezifischer IgE mit einem Gehült von 300 bis 1000 Basophile pro mm³ gebildet wird, erhaltend ausgehend von einer Blutprobe, die einem Patienten entnommen wurde, bei dem man eine Parasitose oder eine Allergie festzustellen versucht, durch Zentrifugieren, um eine Schicht zu erhalten, die im wesentlichen Leukozyten enthält, die mit einem Puffer vermischt wird, der nicht zerstörend auf die basophilen Zellen wirkt, wie insbesondere Hepes-Puffer (N-2-hydroxyethylpiperazin-N'-2-ethansulfonsäure), 1M, oder Pipes-Puffer (Piperazin-N,N''-bis-2-ethansul-fonsäure), worauf das homogenisierte Gemisch auf einer Flüssigkeit mit einer Dichte von 1,079-1,085, deren Osmolarität bei 280 bis 320 Milliosmol liegt, abgelgert wird, zur Bilung, nach dem Zentrifugieren während geeigneter Dauer und bei geeigneter Geschwindigkeit, von einem Ring, der aus einer Suspension besteht, die mit Basophilen angereichert ist, und der eine größere Dichte als die Flüssigkeit aufweist, worauf dieser Ring entnommen und dann vorzugsweise mit dem gleichen Puffer wie vorstehend gewaschen wird, zur Abscheidung in verschiedenen Vertiefungen einer Platte oder eines Kastens zur diagnostischen Ablesung, wobei dieses Verfahren dadurch gekennzeichnet ist, daß man im Verlauf einer ersten Stufe eine Probe des Gesamtblutes, die vorausgehend vorzugsweise in einem Medium von Ethylendiamintetraessigsäure im freien Zustand oder in der Form eines ihrer Salze von einem menschlichen Patienten oder von einem Tier entnommen wurde, von dem angenommen wird, daß er bzw. es von einer oder mehreren Parasitosen oder einer oder mehreren Allergien befallen wurde, die genau Volumen zu Volumen mit einem Hepes-Puffer vermischt wurde, auf einem Medium zur Abtrennung der Basophile abgelagert wird, das hauptsächlich Hepes-Puffer, Polyvinylpyrrolidon und ein wasserlösliches Salz einer Jodbenzolsäure enthält, und dessen Dichte 1,077 - 1,085 beträgt, das anschließend im Verlauf einer zweiten Stufe diese Zusammensetzung bei geeigneter Dauer und Geschwindigkeit zentrifugiert wird, unter Erzielung eines Ringes, der die Basophile und die Lymphozyten enthält, der entnommen und sorgfältig mit Hepes-Puffer gewaschen wird, der eine geeignete Zusammensetzung aufweist und anschlißend verworfen wird, wobei nur der Zellbodensatz behalten wird, der nach der erneuten Suspendierung geeignet ist zur Ablagerung in Vertiefungen einer Platte oder eines Kastens zur diagnostischen Ablesung, wobei in bestimmte davon vorher ein Puffer eingeführt wurde und in bestimmte andere davon vorher Verdünnungen eines oder mehrerer parasitärer Allergene oder Antigene eingebracht wurden.

2. Verfahren nach Anspruch 1, dadurch gekennzeichnet, daß das Trennmedium für die Basophile, das im Verlauf der ersten Stufe des Verfahrens eingesetzt wird, vorzugsweise Heparin, bevorzugt in der Form eines seiner Alkalisalze und besonders bevorzugt seines Natriumsalzes, enthält.

3. Verfahren nach Anspruch 1, dadurch gekennzeichnet, daß das wasserlösliche Salz einer Jodbenzolsäure, das in dem vorstehenden Trennmedium enthalten ist, vorteilhaft aus der Gruppe genommen wird, die wasserlösliche Salze von Trijod- oder Hexajodbenzolsäuren und insbesondere Natrium- und/oder Megluminioxitalamat, Meglumin- und Monoethanolaminioxitalamat, Natrium- und/oder Meglumin-ioxaglat, umfaßt.

4. Verfahren nach Anspruch 1, dadurch gekennzeichnet, daß das Trennmedium für die Basophile aus einer Lösung besteht, die hauptsächlich 3,5 bis 5,5 ml/l Hepes-Puffer, 5 bis 30 g/l Polyvinylpyrrolidon, 220 bis 250 ml/l wasserlösliches Jodbenzolsäuresalz enthält.

5. Verfahren nach Anspruch 1, dadurch gekennzeichnet, daß das Trennmedium für die Basophile vorteilhaft folgende Zusammensetzung aufweist:
Hepes, molare Lösung 3,5 bis 5,5 ml
KCl 10 % 0,49 ml
NaCl 9 % 45 bis 48 ml
Heparin in der Form eines seiner Alkalisalze 2500 - 7500 Einheiten
Polyvinylpyrrolidon 5 - 30 g
wasserlösliches-Jodbenzolsäuresalz 220 - 250 ml
Wasser qsp 1 Liter
Dichte 1,079 - 1,085
pH 7,35 - 7,50 bei 37° C

6. Verfahren zur Herstellung eines Reagenz zur Diagnose von Erkrankungen, die eine Vermehrung spezifischer zirkulierender Immunglobuline und insbesondere von IgE hervorrufen, dadurch gekennzeichnet, daß man eine Suspension weißer Blutkörperchen, herstellt die spezifische IgE tragen, erhalten ausgehend von einer Blutprobe, entnommen von einem menschlichen Patienten oder einem Tier, der bzw. das einem diagnostischen Test unterzogen werden soll, enthält, wobei die Suspension an diesen weißen Blutkörperchen, die spezifische IgE (Basophile) tragen, angereichert ist und erhalten wurde durch Zentrifugieren einer Zusammensetzung, die aus einem Gemisch von genau Volumen zu Volumen der Blutprobe und einem Hepes-Puffer, vorzugsweise heparinisiert, abgelagert auf einem Trennmedium für die weißen Blutkörperchen, das im wesentlichen Hepes-Puffer, Polyvinylpyrrolidon und ein wasserlösliches Salz einer Jodbenzolsäure enthält, und dessen Dichte 1,077-1,085 beträgt, wobei der Zell-Bodensatz der Zentrifugation aus einer Suspension von weißen Blutkörperchen besteht, die spezifische IgE (Basophile) tragen, die das Reagenz zur Diagnose bildet.

7. Verfahren zur Herstellung eines Trennmediumezur Herstellung des diagnostischen Reagenz nach dem vorstehenden Anspruch 5, dadurch gekennzeichnet, daß man hauptsächlich Hepes-Puffer, Polyvinylpyrrolidon und ein wasserlösliches Salz einer Jodbenzolsäure verwendet urd dessen Dichte 1,077-1,085 beträgt.

8. Verfahren zur Herstellung eines Trennmediumsnach dem vorstehenden Anspruch 7, dadurch gekennzeichnet, daß man aus eine Lösung verwendet, die haüptsächlich 3,5 bis 5,5 ml/l Hepes-Puffer, 5 bis 30 g/l Polyvinylpyrrolidon, 220 bis 250 ml/l eines oder mehrerer wasserlöslicher Salze von Jodbenzolsäuren enthält.

9. Verfahren zur Herstellung eines Trennmediumsnach dem vorstehenden Anspruch 8, dadurch gekennzeichnet, daß manvorteilhaft die folgende Zusammensetzung verwendet:

Hepes, molare Lösung 3,5 bis 5,5 ml

KCl 10 % 0,49 ml

NaCl 9 % 45 - 48 ml

Heparin in der Form eines seiner Alkalisalze 2500 - 7500 Einheiter

Polyvinylpyrrolidon 5 - 30 g

wasserlösliches Jodbenzolsäuresalz 220 - 250 ml

wasser qsp 1 Liter

Dichte 1,079 - 1,085

pH 7,35 - 7,50 bei 37° C

10. Verfahren zur Herstellung von Kits, bereit zur Verwendung zur Durchführung des diagnostischen Tests von parasitosen und Allergien, dadurch gekennzeichnet, daß man kombiniert:

- die Gesamtheit der Bestandteile zur Trennung der basophilen Zellen, bestehend aus mehreren geeigneten Rezipienten, wie Ampullen oder Röhrchen, die jeweils eine vordosierte Menge des Trennmediums für die Basophile gemäß dem vorstehenden Anspruch 5 enthalten, sowie mehrere geeignete Rezipienten, wie Ampullen oder Röhrchen, die jeweils eine vordosierte Menge eines Hepes-Puffers mit geeigneter Zusammensetzung zur Wäsche des abgetrennten Lymphozytenringes, abgetrennt durch das Trennmedium, enthalten, wobei der Hepes-Puffer vorteilhaft folgende an sich bekannte Zusammensetzung aufweist:

Hepes-Puffer, molare Lösung 4 - 25 ml

KCl 10 % 0,932 ml

$CaCl_2$ 10 % 0,550 ml

$MgCl_2$ 10 % 0,510 ml

NaCl 9 ‰ 850 - 900 ml

Heparin 10 Einheiten/ml

Wasser q.s.p. 1000 ml

pH 7,4 - 7,6

- und die gesamten Bestandteile zur Durchführung des diagnostischen Tests, bestehend aus mehreren Platten oder Kästen zur diagnostischen Ablesung, die jeweils eine bestimmte Anzahl von Vertiefungen aufweisen, deren Hauptteil, mit Ausnahme der Kontrollvertiefungen, bestimmt ist zur Aufnahme vorbestimmter variabler Mengen eines oder mehrerer Antigene, die spezifisch für eine odere mehrere Allergien oder eine oder mehrere Parasitosen sind, die diagnostisch bestimmt oder aufgespürt werden sollen,

- mehrere Ampullen oder Röhrchen, die vordosierte Mengen einer Lösung zur raschen gleichzeitigen Fixierung und Färbung enthalten,

- ein Röhrchen mit optischem Befestigungsmedium,

- und mehrere Schutzlamellen.

11. Verwendung des diagnostischen Reagenz nach dem vorstehenden Anspruch 5 zur Diagnose einer Parasitose oder einer Allergie zur gleichzeitigen Diagnose verschiedener Parasitosen und/oder Allergien und zur Diagnose anderer Erkrankungen, die eine Vermehrung der spezifischen zirkulierenden Immunglobuline und insbesondere der IgE hervorrufen, wie insbesondere bakterielle, immunpathologische, virale und mykologische Erkrankungen.

**Claims** for the Contracting States BE, CH, DE, GB, LI, LU, NL, SE

1. A process for the preparation of a reagent for diagnosing parasitoses and allergies which cause an increase in the circulating immunoglobulins and especially the IgE's, which diagnostic reagent consists of a suspension of basophilic granulocytes carrying specific IgE's, which contains from 300 to 1000 basophilic cells per $mm^3$ and is obtained from a blood sample taken from a patient who is being examined for a parasitosis or an allergy, the said blood sample being centrifuged in order to collect a layer essentially containing leukocytes, the said layer being mixed with a buffer which is non-destructive towards the basophilic cells, such as 1 M Hepes buffer (N-2-hydroxyethyl-piperazine-N'-ethane-2-sulphonic acid) or Pipes buffer (piperazine-N,N'-bis-ethane-2-sulphonic acid) in particular, the said homogenized mixture then being deposited on a liquid of density 1.079-1.085, the osmolarity of which is between 280 and 320 milliosmoles, so as to give rise, after centrifugation for an appropriate time at an appropriate speed, to a ring which consists of a suspension enriched in basophilic cells and surmounts the liquid of density 1.079-1.085, which ring is removed and then washed, preferably with the same buffer as above, and deposited in the different wells of a diagnostic slide or dish, the said process being characterized in that, in a first step, a sample of whole blood previously taken, preferably in a medium of ethylenediaminetetraacetic acid in the free state or in the form of one of its salts, from a human or animal patient supposedly affected by one or more parasitoses or one or more allergies, and mixed with a substantially equal volume of a Hepes buffer, is deposited on a medium for separating off the basophilic cells, which principally contains Hepes buffer, polyvinylpyrrolidone and a salt of a water-soluble iodoaromatic acid, and whose density is 1.077-1.085, and then in that, in a second step, this composition is centrifuged for an appropriate time at an appropriate speed in order to give a ring containing the basophilic cells and the lymphocytes, the said ring being removed and washed carefully with Hepes buffer of the appropriate composition, this being subsequently discarded so as to leave only the cell residue, which, after resuspension, is ready to be deposited in the wells of a diagnostic slide or dish, buffer having been introduced beforehand into some of these wells and dilutions of parasitic antigen(s) or allergen(s) having been introduced beforehand into other wells.

2. The process according to Claim 1, characterized in that the medium used in the first step of the process for separating off the basophilic cells preferably contains heparin, preferably in the form of one of its alkali metal salts and more particularly its sodium salt.

3. The process according to Claim 1, characterized in that the salt of a water-soluble iodoaromatic acid which is present in the above-mentioned separating medium is advantageously selected from the group comprising salts of water-soluble triiodoaromatic or hexaiodoaromatic acids and more particularly sodium and/or meglumine ioxithalamate, meglumine and monoethanolamine ioxithalamate and sodium and/or meglumine ioxaglate.

4. The process according to Claim 1, characterized in that the said medium for separating off the basophilic cells consists of a solution principally containing from 3.5 to 5.5 ml/litre of Hepes buffer, from 5 to 30 g/litre of polyvinylpyrrolidone and from 220 to 250 ml/litre of a salt of a water-soluble iodoaromatic acid.

5. The process according to Claim 1, characterized in that the said medium for separating off the basophilic cells advantageously has the following composition:

Hepes, molar solution 3.5 to 5.5 ml
KCl, 10% 0.49 ml
NaCl, 9% 45-48 ml
Heparin in the form of one of its alkali metal salts 2500-7500 units
Polyvinylpyrrolidone 5-30 g
Salt of water-soluble iodoaromatic acid 220-250 ml
Water ad 1 litre
Density 1.079-1.085
pH 7.35-7.50 at 37°C

6. A reagent for diagnosing diseases which cause an increase in the circulating specific immunoglobulins and especially the IgE's, characterized in that it consists of a suspension of white blood cells carrying specific IgE's, which is obtained from a blood sample taken from a human or animal patient who is to undergo a diagnostic test, which suspension is enriched in the said white blood cells carrying specific IgE's (basophilic cells) and is obtained by centrifugation of a composition consisting of a mixture of substantially equal volumes of the said blood sample and a Hepes buffer preferably containing heparin, which is deposited on a medium for separating off the said white blood cells, the said medium essentially comprising Hepes buffer, polyvinylpyrrolidone and a salt of a watersoluble iodoaromatic acid and having a density of 1.077-1.085, the cell residue resulting from the said centrifugation consisting of the suspension of white blood cells carrying specific IgE's (basophilic cells) which forms the diagnostic reagent.

7. A separating medium for preparing the diagnostic reagent, according to Claim 5 above, characterized in that it principally contains Hepes buffer, polyvinylpyrrolidone and a salt of a water-soluble iodoaromatic acid and has a density of 1.077-1.085.

8. The separating medium according to Claim 7 above, characterized in that it consists of a solution principally containing from 3.5 to 5.5 ml/litre of Hepes buffer, from 5 to 30 g/litre of polyvinylpyrrolidone and from 220 to 250 ml/litre of one or more salts of water-soluble iodoaromatic acids.

9. The separating medium according to Claim 8 above, characterized in that it advantageously has the following composition.:

Hepes, molar solution 3.5 to 5.5 ml
KCl, 10% 0.49 ml
NaCl, 9% 45-48 ml
Heparin in the form of one of its alkali metal salts 2500-7500 units
Polyvinylpyrrolidone 5-30 g
Salt of water-soluble iodoaromatic acid 220-250 ml
Water ad 1 litre
Density 1.079-1.085
pH 7.35-7.50 at 37°C

10. Ready-to-use kits for carrying out the test for diagnosing parasitoses and allergies, characterized in that they comprise:

- a set of components for separating off the basophilic cells, consisting of several appropriate receptacles, such as ampoules or tubes, each containing a predetermined quantity of the medium for separating off the basophilic cells, according to Claim 5 above, and several appropriate receptacles, such as ampoules or tubes, each containing a predetermined quantity of a Hepes buffer of appropriate composition for washing the lymphocyte ring separated off by the separating medium, which Hepes buffer advantageously has the

14

following composition known per se:

Hepes buffer, molar solution 4 to 25 ml

KCl, 10% 0.932 ml

$CaCl_2$ 10% 0.550 ml

$MgCl_2$ 10% 0.510 ml

NaCl, 9‰. 850 to 900 ml

Heparin 10 units/ml

Water ad 1000 ml

pH 7.4-7.6

- and a set of components for carrying out the diagnostic test, consisting of several diagnostic slides or dishes each having a number of wells, most of which, excepting the reference wells, are intended to receive variable predetermined quantities of one or more antigens specific for one or more allergies or one or more parasitoses to be diagnosed or detected, several ampoules or tubes containing predetermined quantities of a solution for rapid and simultaneous fixing and staining, a tube of optical mounting medium and several cover glasses.

11. Application of the diagnostic reagent according to Claim 5 above to the diagnosis of a parasitosis or an allergy, the simultaneous diagnosis of several parasitoses and/or allergies and the diagnosis of other diseases which cause an increase in the circulating specific immunoglobulins and especially the IgE's, such as, in particular, bacterial, immunopathological, viral and mycological diseases.

**Claims** for the Contracting States AT

1. A process for the preparation of a reagent for diagnosing parasitoses and allergies which cause an increase in the circulating immunoglobulins and especially the IgE's, which diagnostic reagent consists of a suspension of basophilic granulocytes carrying specific IgE's, which contains from 300 to 1000 basophilic cells per $mm^3$ and is obtained from a blood sample taken from a patient who is being examined for a parasitosis or an allergy, the said blood sample being centrifuged in order to collect a layer essentially containing leukocytes, the said layer being mixed with a buffer which is non-destructive towards the basophilic cells, such as 1 M Hepes buffer (N-2-hydroxyethyl-piperazine-N'-ethane-2-sulphonic acid) or Pipes buffer (piperazine-N,N'-bis-ethane-2-sulphonic acid) in particular, the said homogenized mixture then being deposited on a liquid of density 1.079-1.085, the osmolarity of which is between 280 and 320 milliosmoles, so as to give rise, after centrifugation for an appropriate time at an appropriate speed, to a ring which consists of a suspension enriched in basophilic cells and surmounts the liquid of density 1.079-1.085, which ring is removed and then washed, preferably with the same buffer as above, and deposited in the different wells of a diagnostic slide or dish, the said process being characterized in that, in a first step, a sample of whole blood previously taken, preferably in a medium of ethylenediaminetetraacetic acid in the free state or in the form of one of its salts, from a human or animal patient supposedly affected by one or more parasitoses or one or more allergies, and mixed with a substantially equal volume of a Hepes buffer, is deposited on a medium for separating off the basophilic cells, which principally contains Hepes buffer, polyvinylpyrrolidone and a salt of a water-soluble iodoaromatic acid, and whose density is 1.077-1.085, and then in that, in a second step, this composition is centrifuged for an appropriate time at an appropriate speed in order to give a ring containing the basophilic cells and the lymphocytes, the said ring being removed and washed carefully with Hepes buffer of the appropriate composition, this being subsequently discarded so as to leave only the cell residue, which, after resuspension, is ready to be deposited in the wells of a diagnostic slide or dish, buffer having been introduced beforehand into some of these wells and dilutions of parasitic antigen(s) or allergen(s) having been introduced beforehand into other wells.

2. The process according to Claim 1, characterized in that the medium used in the first step of the process for separating off the basophilic cells preferably contains heparin, preferably in the form of one of its alkali metal salts and more particularly its sodium salt.

3. The process according to Claim 1, characterized in that the salt of a water-soluble iodoaromatic acid which is present in the above-mentioned separating medium is advantageously selected from the group comprising salts of water-soluble triiodoaromatic or hexaiodoaromatic acids and more particularly sodium and/or meglumine ioxithalamate, meglumine and monoethanolamine ioxithalamate and sodium and/or meglumine ioxaglate.

4. The process according to Claim 1, characterized in that the said medium for separating off the basophilic cells consists of a solution principally containing from 3.5 to 5.5 ml/litre of Hepes buffer, from 5 to 30 g/litre of polyvinylpyrrolidone and from 220 to 250 ml/litre of a salt of a water-soluble iodoaromatic acid.

5. The process according to Claim 1, characterized in that the said medium for separating off the basophilic cells advantageously has the following composition:

Hepes, molar solution 3.5 to 5.5 ml

KCl, 10% 0.49 ml

NaCl, 9% 45-48 ml

Heparin in the form of one of its alkali metal salts 2500-7500 units

15

Polyvinylpyrrolidone 5-30 g
Salt of water-soluble iodoaromatic acid 220-250 ml
Water ad 1 litre
Density 1.079-1.085
pH 7.35-7.50 at 37°C

6. A process for the preparation of a reagent for diagnosing diseases which cause an increase in the circulating specific immunoglobulines and especially the IgE's, characterized in that the said reagent consists of a suspension of white blood cells carrying specific IgE's, which is obtained from a blood sample taken from a human or animal patient who is to undergo a diagnostic test, which suspension is enriched in the said white blood cells carrying specific IgE's (basophilic cells) and is obtained by centrifugation of a composition consisting of a mixture of substantially equal volumes of the said blood sample and a Hepes buffer preferably containing heparin, which is deposited on a medium for separating off the said white blood cells, the said medium essentially comprising Hepes buffer, polyvinylpyrrolidone and a salt of a water-soluble iodoaromatic acid and having a density of 1.077-1.085, the cell residue resulting from the said centrifugation consisting of the suspension of white blood cells carrying specific IgE's (basophilic cells) which forms the diagnostic reagent.

7. A process for the preparation of a separating medium for preparing the diagnostic reagent, according to Claim 5 above, characterized in that the said medium principally contains Hepes buffer, polyvinylpyrrolidone and a salt of a water-soluble iodoaromatic acid and has a density of 1.077-1.085.

8. A process for the preparation of a separating medium according to Claim 7 above, characterized in that the said medium consists of a solution principally containing from 3.5 to 5.5 ml/litre of Hepes buffer, from 5 to 30 g/litre of polyvinylpyrrolidone and from 220 to 250 ml/litre of one or more salts of water-soluble iodoaromatic acids.

9. A process for the preparation of a separating medium according to Claim 8 above, characterized in that the said medium advantageously has the following composition:

Hepes, molar solution 3.5 to 5.5 ml
KCl, 10% 0.49 ml
NaCl, 9% 45-48 ml
Heparin in the form of one of its alkali metal salts 2500-7500 units
Polyvinylpyrrolidone 5-30 g
Salt of water-soluble iodoaromatic acid 220-250 ml
Water ad 1 litre
Density 1.079-1.085
pH 7.35-7.50 at 37°C

10. A process for the preparation of ready-to-use kits for carrying out the test for diagnosing parasitoses and allergies, characterized in that the said kits comprise:

- a set of components for separating off the basophilic cells, consisting of several appropriate receptacles, such as ampoules or tubes, each containing a predetermined quantity of the medium for separating off the basophilic cells, according to Claim 5 above, and several appropriate receptacles, such as ampoules or tubes, each containing a predetermined quantity of a Hepes buffer of appropriate composition for washing the lymphocyte ring separated off by the separating medium, which Hepes buffer advantageously has the following composition known per se:

Hepes buffer, molar solution 4 to 25 ml
KCl, 10% 0.932 ml
CaCl$_2$, 10% 0.550 ml
MgCl$_2$, 10% 0.510 ml
NaCl, 9‰ 850 to 900 ml
Heparin 10 units/ml
Water ad 1000 ml
pH 7.4-7.6

- and a set of components for carrying out the diagnostic test, consisting of several diagnostic slides or dishes each having a number of wells, most of which, excepting the reference wells, are intended to receive variable predetermined quantities of one or more antigens specific for one or more allergies or one or more parasitoses to be diagnosed or detected, several ampoules or tubes containing predetermined quantities of a solution for rapid and simultaneous fixing and staining, a tube of optical mounting medium and several cover glasses.

11. Application of the diagnostic reagent according to Claim 5 above to the diagnosis of a parasitosis or an allergy, the simultaneous diagnosis of several parasitoses and/or allergies and the diagnosis of other diseases which cause an increase in the circulating specific immunoglobulins and especially the IgE's, such as, in particular, bacterial, immunopathological, viral and mycological diseases.